# EUROPEAN PATENT SPECIFICATION

(11) **EP 1 548 113 B1**
(45) Date of publication and mention of the grant of the patent: **24.01.2007**
(21) Application number: 04028520.7
(22) Date of filing: 02.12.2004
(51) Int. Cl.: C12N 15/10, C12Q 1/68

(54) **A method for obtaining circular mutated and/or chimaeric polynucleotides**
Verfahren zur Herstellung von zirkulären mutierten und/oder chimären Polynukleotiden
Procédé pour l'obtention de polynucléotides circulaires mutés et/ou chimériques

(30) Priority: 04.12.2003 EP 03027838
(43) Date of publication of application: 29.06.2005
(73) Proprietor: Roche Diagnostics GmbH, 68305 Mannheim (DE); F.HOFFMANN-LA ROCHE AG, 4070 Basel (CH)
(72) Inventor: Kratzsch, Peter, 82387 Antdorf (DE); Von Der Eltz, Herbert Dr., 82362 Weilheim (DE); Schmuck, Rainer Dr., 83671 Benediktbeuern (DE); Boenitz-Dulat, Mara Dr., D- 82327 Tutzing (DE)

(56) References cited:
- WO-A-01/33944
- WO-A-02/24953
- WO-A-99/29902
- VOLKOV A A ET AL: "RANDOM CHIMERAGENESIS BY HETERODUPLEX RECOMBINATION" METHODS IN ENZYMOLOGY, ACADEMIC PRESS INC, SAN DIEGO, CA, US, vol. 328, 2000, pages 456-463, XP000990804 ISSN: 0076-6879
- SHAO ZHIXIN ET AL: "Random-priming in vitro recombination: An effective tool for directed evolution" NUCLEIC ACIDS RESEARCH, OXFORD UNIVERSITY PRESS, SURREY, GB, vol. 26, no. 2, 15 January 1998 (1998-01-15), pages 681-683, XP002162556 ISSN: 0305-1048

### SEQUENCE LISTING

<110> Roche Diagnostics GmbH
   F. Hoffmann-La Roche AG
<120> A method for obtaining circular mutated and/or chimaeric polynucleotides
<130> 22312 EP1
<150> EP03027838.6
   <151> 2004-12-04
<160> 9
<170> PatentIn version 3.2
<210> 1
   <211> 1533
   <212> DNA
   <213> Bos taurus
<220>
   <221> CDS
   <222> (1)..(1533)
   <223> DNA sequence coding for a calf intestinal alkaline phosphatase (ciAP)
<400> 1
   atg aat aag aag gta ctg acc ctt tct gcc gtg atg gca agt ctg tta      48
   Met Asn Lys Lys Val Leu Thr Leu Ser Ala Val Met Ala Ser Leu Leu
   1               5                   10                  15
   ttc ggg gcc cac gcg cac gcg gcg atc cca gct gag gag gaa aac ccc      96
   Phe Gly Ala His Ala His Ala Ala Ile Pro Ala Glu Glu Glu Asn Pro
               20                  25                  30
   gcc ttc tgg aac cgc cag gca gcc cag gcc ctt gat gta gcc aag aag     144
   Ala Phe Trp Asn Arg Gln Ala Ala Gln Ala Leu Asp Val Ala Lys Lys
           35                  40                  45
   ttg cag ccg atc cag aca gct gcc aag aat gtc atc ctc ttc ttg ggg     192
   Leu Gln Pro Ile Gln Thr Ala Ala Lys Asn Val Ile Leu Phe Leu Gly
       50                  55                  60
   gat ggg atg ggg gtg cct acg gtg aca gcc act cgg atc cta aag ggg     240
   Asp Gly Met Gly Val Pro Thr Val Thr Ala Thr Arg Ile Leu Lys Gly
   65                  70                  75                  80
   cag atg aat ggc aaa ctg gga cct gag aca ccc ctg gcc atg gac cag     288
   Gln Met Asn Gly Lys Leu Gly Pro Glu Thr Pro Leu Ala Met Asp Gln
                   85                  90                  95
   ttc cca tac gtg gct ctg tcc aag aca tac aac gtg gac aga cag gtg     336
   Phe Pro Tyr Val Ala Leu Ser Lys Thr Tyr Asn Val Asp Arg Gln Val
               100                 105                 110
   cca gac agc gca ggc act gcc act gcc tac ctg tgt ggg gtc aag ggc      384
   Pro Asp Ser Ala Gly Thr Ala Thr Ala Tyr Leu Cys Gly Val Lys Gly
           115                 120                 125
   aac tac aga acc atc ggt gta agt gca gcc gcc cgc tac aat cag tgc      432
   Asn Tyr Arg Thr Ile Gly Val Ser Ala Ala Ala Arg Tyr Asn Gln Cys
       130                 135                 140
   aac acg aca cgt ggg aat gag gtc acg tct gtg atc aac cgg gcc aag      480
   Asn Thr Thr Arg Gly Asn Glu Val Thr Ser Val Ile Asn Arg Ala Lys
   145                 150                 155                 160
   aaa gca ggg aag gcc gtg gga gtg gtg acc acc acc agg gtg cag cat      528
   Lys Ala Gly Lys Ala Val Gly Val Val Thr Thr Thr Arg Val Gln His
                   165                 170                 175
   gcc tcc cca gcc ggg gcc tac gcg cac acg gtg aac cga aac tgg tac      576
   Ala Ser Pro Ala Gly Ala Tyr Ala His Thr Val Asn Arg Asn Trp Tyr
               180                 185                 190
   tca gac gcc gac ctg cct gct gat gca cag aag aat ggc tgc cag gac      624
   Ser Asp Ala Asp Leu Pro Ala Asp Ala Gln Lys Asn Gly Cys Gln Asp
           195                 200                 205
   atc gcc gca cag ctg gtc tac aac atg gat att gac gtg atc ctg ggt      672
   Ile Ala Ala Gln Leu Val Tyr Asn Met Asp Ile Asp Val Ile Leu Gly
       210                 215                 220
   gga ggc cga atg tac atg ttt cct gag ggg acc cca gac cct gaa tac      720
   Gly Gly Arg Met Tyr Met Phe Pro Glu Gly Thr Pro Asp Pro Glu Tyr
   225                 230                 235                 240
   cca gat gat gcc agt gtg aat gga gtc cgg aag gac aag cag aac ctg      768
   Pro Asp Asp Ala Ser Val Asn Gly Val Arg Lys Asp Lys Gln Asn Leu
                   245                 250                 255
   gtg cag gaa tgg cag gcc aag cac cag gga gcc cag tat gtg tgg aac      816
   Val Gln Glu Trp Gln Ala Lys His Gln Gly Ala Gln Tyr Val Trp Asn
               260                 265                 270
   cgc act gcg ctc ctt cag gcg gcc gat gac tcc agt gta aca cac ctc      864
   Arg Thr Ala Leu Leu Gln Ala Ala Asp Asp Ser Ser Val Thr His Leu
           275                 280                 285
   atg ggc ctc ttt gag ccg gca gac atg aag tat aat gtt cag caa gac      912
   Met Gly Leu Phe Glu Pro Ala Asp Met Lys Tyr Asn Val Gln Gln Asp
       290                 295                 300
   cac acc aag gac ccg acc ctg gcg gag atg acg gag gcg gcc ctg caa      960
   His Thr Lys Asp Pro Thr Leu Ala Glu Met Thr Glu Ala Ala Leu Gln
   305                 310                 315                 320
   gtg ctg agc agg aac ccc cgg ggc ttc tac ctc ttc gtg gag gga ggc     1008
   Val Leu Ser Arg Asn Pro Arg Gly Phe Tyr Leu Phe Val Glu Gly Gly
                   325                 330                 335
   cgc att gac cac ggt cac cat gac ggc aaa gct tat atg gca ctg act      1056
   Arg Ile Asp His Gly His His Asp Gly Lys Ala Tyr Met Ala Leu Thr
               340                 345                 350
   gag gcg atc atg ttt gac aat gcc atc gcc aag gct aac gag ctc act      1104
   Glu Ala Ile Met Phe Asp Asn Ala Ile Ala Lys Ala Asn Glu Leu Thr
           355                 360                 365
   agc gaa ctg gac acg ctg atc ctt gtc act gca gac cac tcc cat gtc      1152
   Ser Glu Leu Asp Thr Leu Ile Leu Val Thr Ala Asp His Ser His Val
       370                 375                 380
   ttc tcc ttt ggt ggc tac aca ctg cgt ggg acc tcc att ttc ggt ctg      1200
   Phe Ser Phe Gly Gly Tyr Thr Leu Arg Gly Thr Ser Ile Phe Gly Leu
   385                 390                 395                 400
   gcc ccc ggc aag gcc tta gac agc aag tcc tac acc tcc atc ctc tat      1248
   Ala Pro Gly Lys Ala Leu Asp Ser Lys Ser Tyr Thr Ser Ile Leu Tyr
                   405                 410                 415
   ggc aat ggc cca ggc tat gcg ctt ggc ggg ggc tcg agg ccc gat gtt      1296
   Gly Asn Gly Pro Gly Tyr Ala Leu Gly Gly Gly Ser Arg Pro Asp Val
               420                 425                 430
   aat ggc agc aca agc gag gaa ccc tcg tac cgg cag cag gcg gcc gtg      1344
   Asn Gly Ser Thr Ser Glu Glu Pro Ser Tyr Arg Gln Gln Ala Ala Val
           435                 440                 445
   ccc ctg gct agc gag acc cac ggg ggc gaa gac gtg gcg gtg ttc gcg      1392
   Pro Leu Ala Ser Glu Thr His Gly Gly Glu Asp Val Ala Val Phe Ala
       450                 455                 460
   cga ggc ccg cag gcg cac ctg gtg cac ggc gtg cag gag gag acc ttc      1440
   Arg Gly Pro Gln Ala His Leu Val His Gly Val Gln Glu Glu Thr Phe
   465                 470                 475                 480
   gtg gcg cac atc atg gcc ttt gcg ggc tgc gtg gag ccc tac acc gac      1488
   Val Ala His Ile Met Ala Phe Ala Gly Cys Val Glu Pro Tyr Thr Asp
                   485                 490                 495
   tgc aat ctg cca gcc ccc tcc acc gcc acc agc atc ccc gac tga          1533
   Cys Asn Leu Pro Ala Pro Ser Thr Ala Thr Ser Ile Pro Asp
               500                 505                 510
<210> 2
   <211> 510
   <212> PRT
   <213> Bos taurus
<400> 2
   Met Asn Lys Lys Val Leu Thr Leu Ser Ala Val Met Ala Ser Leu Leu
   1               5                   10                  15
   Phe Gly Ala His Ala His Ala Ala Ile Pro Ala Glu Glu Glu Asn Pro
               20                  25                  30
   Ala Phe Trp Asn Arg Gln Ala Ala Gln Ala Leu Asp Val Ala Lys Lys
           35                  40                  45
   Leu Gln Pro Ile Gln Thr Ala Ala Lys Asn Val Ile Leu Phe Leu Gly
       50                  55                  60
   Asp Gly Met Gly Val Pro Thr Val Thr Ala Thr Arg Ile Leu Lys Gly
   65                  70                  75                  80
   Gln Met Asn Gly Lys Leu Gly Pro Glu Thr Pro Leu Ala Met Asp Gln
                   85                  90                  95
   Phe Pro Tyr Val Ala Leu Ser Lys Thr Tyr Asn Val Asp Arg Gln Val
               100                 105                 110
   Pro Asp Ser Ala Gly Thr Ala Thr Ala Tyr Leu Cys Gly Val Lys Gly
           115                 120                 125
   Asn Tyr Arg Thr Ile Gly Val Ser Ala Ala Ala Arg Tyr Asn Gln Cys
       130                 135                 140
   Asn Thr Thr Arg Gly Asn Glu Val Thr Ser Val Ile Asn Arg Ala Lys
   145                 150                 155                 160
   Lys Ala Gly Lys Ala Val Gly Val Val Thr Thr Thr Arg Val Gln His
                   165                 170                 175
   Ala Ser Pro Ala Gly Ala Tyr Ala His Thr Val Asn Arg Asn Trp Tyr
               180                 185                 190
   Ser Asp Ala Asp Leu Pro Ala Asp Ala Gln Lys Asn Gly Cys Gln Asp
           195                 200                 205
   Ile Ala Ala Gln Leu Val Tyr Asn Met Asp Ile Asp Val Ile Leu Gly
       210                 215                 220
   Gly Gly Arg Met Tyr Met Phe Pro Glu Gly Thr Pro Asp Pro Glu Tyr
   225                 230                 235                 240
   Pro Asp Asp Ala Ser Val Asn Gly Val Arg Lys Asp Lys Gln Asn Leu
                   245                 250                 255
   Val Gln Glu Trp Gln Ala Lys His Gln Gly Ala Gln Tyr Val Trp Asn
               260                 265                 270
   Arg Thr Ala Leu Leu Gln Ala Ala Asp Asp Ser Ser Val Thr His Leu
           275                 280                 285
   Met Gly Leu Phe Glu Pro Ala Asp Met Lys Tyr Asn Val Gln Gln Asp
       290                 295                 300
   His Thr Lys Asp Pro Thr Leu Ala Glu Met Thr Glu Ala Ala Leu Gln
   305                 310                 315                 320
   Val Leu Ser Arg Asn Pro Arg Gly Phe Tyr Leu Phe Val Glu Gly Gly
                   325                 330                 335
   Arg Ile Asp His Gly His His Asp Gly Lys Ala Tyr Met Ala Leu Thr
               340                 345                 350
   Glu Ala Ile Met Phe Asp Asn Ala Ile Ala Lys Ala Asn Glu Leu Thr
           355                 360                 365
   Ser Glu Leu Asp Thr Leu Ile Leu Val Thr Ala Asp His Ser His Val
       370                 375                 380
   Phe Ser Phe Gly Gly Tyr Thr Leu Arg Gly Thr Ser Ile Phe Gly Leu
   385                 390                 395                 400
   Ala Pro Gly Lys Ala Leu Asp Ser Lys Ser Tyr Thr Ser Ile Leu Tyr
                   405                 410                 415
   Gly Asn Gly Pro Gly Tyr Ala Leu Gly Gly Gly Ser Arg Pro Asp Val
               420                 425                 430
   Asn Gly Ser Thr Ser Glu Glu Pro Ser Tyr Arg Gln Gln Ala Ala Val
           435                 440                 445
   Pro Leu Ala Ser Glu Thr His Gly Gly Glu Asp Val Ala Val Phe Ala
       450                 455                 460
   Arg Gly Pro Gln Ala His Leu Val His Gly Val Gln Glu Glu Thr Phe
   465 470 475 480
   Val Ala His Ile Met Ala Phe Ala Gly Cys Val Glu Pro Tyr Thr Asp
   485 490 495
   Cys Asn Leu Pro Ala Pro Ser Thr Ala Thr Ser Ile Pro Asp
   500 505 510
<210> 3
   <211> 1527
   <212> DNA
   <213> Homo sapiens
<220>
   <221> CDS
   <222> (1)..(1527)
   <223> DNA sequence coding for a human placental alkaline phosphatase (hpAP)
<400> 3
   atg aat aag aag gta ctg acc ctt tct gcc gtg atg gca agt ctg tta 48
   Met Asn Lys Lys Val Leu Thr Leu Ser Ala Val Met Ala Ser Leu Leu
   1 5 10 15
   ttc ggc gcg cac gcg cac gcg gcg atc cca gtt gag gag gag aac ccg 96
   Phe Gly Ala His Ala His Ala Ala Ile Pro Val Glu Glu Glu Asn Pro
   20 25 30
   gac ttc tgg aac cgc gag gca gcc gag gcc ctg ggt gcc gcc aag aag 144
   Asp Phe Trp Asn Arg Glu Ala Ala Glu Ala Leu Gly Ala Ala Lys Lys
   35 40 45
   ctg cag cct gca cag aca gcc gcc aag aac ctc atc atc ttc ctg ggc 192
   Leu Gln Pro Ala Gln Thr Ala Ala Lys Asn Leu Ile Ile Phe Leu Gly
   50 55 60
   gat ggg atg ggg gtg tct acg gtg aca gct gcc agg atc cta aaa ggg 240
   Asp Gly Met Gly Val Ser Thr Val Thr Ala Ala Arg Ile Leu Lys Gly
   65 70 75 80
   cag aag aag gac aaa ctg ggg cct gag ata ccc ctg gcc atg gac cgc 288
   Gln Lys Lys Asp Lys Leu Gly Pro Glu Ile Pro Leu Ala Met Asp Arg
   85 90 95
   ttc cca tat gtg gct ctg tcc aag aca tac aat gta gac aaa cat gtg 336
   Phe Pro Tyr Val Ala Leu Ser Lys Thr Tyr Asn Val Asp Lys His Val
   100 105 110
   cca gac agt gga gcc aca gcc acg gcc tac ctg tgc ggg gtc aag ggc      384
   Pro Asp Ser Gly Ala Thr Ala Thr Ala Tyr Leu Cys Gly Val Lys Gly
           115                 120                 125
   aac ttc cag acc att ggc ttg agt gca gcc gcc cgc ttt aac cag tgc      432
   Asn Phe Gln Thr Ile Gly Leu Ser Ala Ala Ala Arg Phe Asn Gln Cys
       130                 135                 140
   aac acg aca cgc ggc aac gag gtc atc tcc gtg atg aat cgg gcc aag      480
   Asn Thr Thr Arg Gly Asn Glu Val Ile Ser Val Met Asn Arg Ala Lys
   145                 150                 155                 160
   aaa gca ggg aag tca gtg gga gtg gta acc acc aca cga gtg cag cac      528
   Lys Ala Gly Lys Ser Val Gly Val Val Thr Thr Thr Arg Val Gln His
                   165                 170                 175
   gcc tcg cca gcc ggc acc tac gcc cac acg gtg aac cgc aac tgg tac      576
   Ala Ser Pro Ala Gly Thr Tyr Ala His Thr Val Asn Arg Asn Trp Tyr
               180                 185                 190
   tcg gac gcc gac gtg cct gcc tcg gcc cgc cag gag ggg tgc cag gac      624
   Ser Asp Ala Asp Val Pro Ala Ser Ala Arg Gln Glu Gly Cys Gln Asp
           195                 200                 205
   atc gct acg cag ctc atc tcc aac atg gac att gac gtg atc cta ggt      672
   Ile Ala Thr Gln Leu Ile Ser Asn Met Asp Ile Asp Val Ile Leu Gly
       210                 215                 220
   gga ggc cga aag tac atg ttt ccc atg gga acc cca gac cct gag tac      720
   Gly Gly Arg Lys Tyr Met Phe Pro Met Gly Thr Pro Asp Pro Glu Tyr
   225                 230                 235                 240
   cca gat gac tac agc caa ggt ggg acc agg ctg gac ggg aag aat ctg      768
   Pro Asp Asp Tyr Ser Gln Gly Gly Thr Arg Leu Asp Gly Lys Asn Leu
                   245                 250                 255
   gtg cag gaa tgg ctg gcg aag cgc cag ggt gcc cgg tat gtg tgg aac      816
   Val Gln Glu Trp Leu Ala Lys Arg Gln Gly Ala Arg Tyr Val Trp Asn
               260                 265                 270
   cgc act gag ctc atg cag gct tcc ctg gac ccg tct gtg acc cat ctc      864
   Arg Thr Glu Leu Met Gln Ala Ser Leu Asp Pro Ser Val Thr His Leu
           275                 280                 285
   atg ggt ctc ttt gag cct gga gac atg aaa tac gag atc cac cga gac      912
   Met Gly Leu Phe Glu Pro Gly Asp Met Lys Tyr Glu Ile His Arg Asp
       290                 295                 300
   tcc aca ctg gac ccc tcc ctg atg gag atg aca gag gct gcc ctg cgc      960
   Ser Thr Leu Asp Pro Ser Leu Met Glu Met Thr Glu Ala Ala Leu Arg
   305                 310                 315                 320
   ctg ctg agc agg aac ccc cgc ggc ttc ttc ctc ttc gtg gag ggt ggt     1008
   Leu Leu Ser Arg Asn Pro Arg Gly Phe Phe Leu Phe Val Glu Gly Gly
                   325                 330                 335
   cgc atc gac cat ggt cat cat gaa agc agg gct tac cgg gca ctg act      1056
   Arg Ile Asp His Gly His His Glu Ser Arg Ala Tyr Arg Ala Leu Thr
               340                 345                     350
   gag acg atc atg ttc gac gac gcc att gag agg gcg ggc cag ctc acc      1104
   Glu Thr Ile Met Phe Asp Asp Ala Ile Glu Arg Ala Gly Gln Leu Thr
           355                 360                     365
   agc gag gag gac acg ctg agc ctc gtc act gcc gac cac tcc cac gtc      1152
   Ser Glu Glu Asp Thr Leu Ser Leu Val Thr Ala Asp His Ser His Val
       370                 375                     380
   ttc tcc ttc gga ggc tac ccc ctg cga ggg agc tcc atc ttc ggg ctg      1200
   Phe Ser Phe Gly Gly Tyr Pro Leu Arg Gly Ser Ser Ile Phe Gly Leu
   385                 390                     395                 400
   gcc cct ggc aag gcc cgg gac agg aag gcc tac acg gtc ctc cta tac      1248
   Ala Pro Gly Lys Ala Arg Asp Arg Lys Ala Tyr Thr Val Leu Leu Tyr
                   405                 410                     415
   gga aac ggt cca ggc tat gtg ctc aag gac ggc gcc cgg ccg gat gtt      1296
   Gly Asn Gly Pro Gly Tyr Val Leu Lys Asp Gly Ala Arg Pro Asp Val
               420                 425                     430
   acc gag agc gag agc ggg agc ccc gag tat cgg cag cag tca gca gtg      1344
   Thr Glu Ser Glu Ser Gly Ser Pro Glu Tyr Arg Gln Gln Ser Ala Val
           435                 440                     445
   ccc ctg gac gaa gag acc cac gca ggc gag gac gtg gcg gtg ttc gcg      1392
   Pro Leu Asp Glu Glu Thr His Ala Gly Glu Asp Val Ala Val Phe Ala
       450                 455                     460
   cgc ggc ccg cag gcg cac ctg gtt cac ggc gtg cag gag cag acc ttc      1440
   Arg Gly Pro Gln Ala His Leu Val His Gly Val Gln Glu Gln Thr Phe
   465                 470                     475                 480
   ata gcg cac gtc atg gcc ttc gcc gcc tgc ctg gag ccc tac acc gcc      1488
   Ile Ala His Val Met Ala Phe Ala Ala Cys Leu Glu Pro Tyr Thr Ala
                   485                 490                     495
   tgc gac ctg gcg ccc ccc gcc ggc acc acc gac tga taa                  1527
   Cys Asp Leu Ala Pro Pro Ala Gly Thr Thr Asp
               500                 505
<210> 4
   <211> 507
   <212> PRT
   <213> Homo sapiens
<400> 4
   Met Asn Lys Lys Val Leu Thr Leu Ser Ala Val Met Ala Ser Leu Leu
   1               5                   10                  15
   Phe Gly Ala His Ala His Ala Ala Ile Pro Val Glu Glu Glu Asn Pro
               20                  25                  30
   Asp Phe Trp Asn Arg Glu Ala Ala Glu Ala Leu Gly Ala Ala Lys Lys
           35                  40                  45
   Leu Gln Pro Ala Gln Thr Ala Ala Lys Asn Leu Ile Ile Phe Leu Gly
       50                  55                  60
   Asp Gly Met Gly Val Ser Thr Val Thr Ala Ala Arg Ile Leu Lys Gly
   65                  70                  75                  80
   Gln Lys Lys Asp Lys Leu Gly Pro Glu Ile Pro Leu Ala Met Asp Arg
                   85                  90                  95
   Phe Pro Tyr Val Ala Leu Ser Lys Thr Tyr Asn Val Asp Lys His Val
               100                 105                 110
   Pro Asp Ser Gly Ala Thr Ala Thr Ala Tyr Leu Cys Gly Val Lys Gly
           115                 120                 125
   Asn Phe Gln Thr Ile Gly Leu Ser Ala Ala Ala Arg Phe Asn Gln Cys
       130                 135                 140
   Asn Thr Thr Arg Gly Asn Glu Val Ile Ser Val Met Asn Arg Ala Lys
   145                 150                 155                 160
   Lys Ala Gly Lys Ser Val Gly Val Val Thr Thr Thr Arg Val Gln His
                   165                 170                 175
   Ala Ser Pro Ala Gly Thr Tyr Ala His Thr Val Asn Arg Asn Trp Tyr
               180                 185                 190
   Ser Asp Ala Asp Val Pro Ala Ser Ala Arg Gln Glu Gly Cys Gln Asp
           195                 200                 205
   Ile Ala Thr Gln Leu Ile Ser Asn Met Asp Ile Asp Val Ile Leu Gly
       210                 215                 220
   Gly Gly Arg Lys Tyr Met Phe Pro Met Gly Thr Pro Asp Pro Glu Tyr
   225                 230                 235                 240
   Pro Asp Asp Tyr Ser Gln Gly Gly Thr Arg Leu Asp Gly Lys Asn Leu
                   245                 250                 255
   Val Gln Glu Trp Leu Ala Lys Arg Gln Gly Ala Arg Tyr Val Trp Asn
   260 265 270
   Arg Thr Glu Leu Met Gln Ala Ser Leu Asp Pro Ser Val Thr His Leu
   275 280 285
   Met Gly Leu Phe Glu Pro Gly Asp Met Lys Tyr Glu Ile His Arg Asp
   290 295 300
   Ser Thr Leu Asp Pro Ser Leu Met Glu Met Thr Glu Ala Ala Leu Arg
   305 310 315 320
   Leu Leu Ser Arg Asn Pro Arg Gly Phe Phe Leu Phe Val Glu Gly Gly
   325 330 335
   Arg Ile Asp His Gly His His Glu Ser Arg Ala Tyr Arg Ala Leu Thr
   340 345 350
   Glu Thr Ile Met Phe Asp Asp Ala Ile Glu Arg Ala Gly Gln Leu Thr
   355 360 365
   Ser Glu Glu Asp Thr Leu Ser Leu Val Thr Ala Asp His Ser His Val
   370 375 380
   Phe Ser Phe Gly Gly Tyr Pro Leu Arg Gly Ser Ser Ile Phe Gly Leu
   385 390 395 400
   Ala Pro Gly Lys Ala Arg Asp Arg Lys Ala Tyr Thr Val Leu Leu Tyr
   405 410 415
   Gly Asn Gly Pro Gly Tyr Val Leu Lys Asp Gly Ala Arg Pro Asp Val
   420 425 430
   Thr Glu Ser Glu Ser Gly Ser Pro Glu Tyr Arg Gln Gln Ser Ala Val
   435 440 445
   Pro Leu Asp Glu Glu Thr His Ala Gly Glu Asp Val Ala Val Phe Ala
   450 455 460
   Arg Gly Pro Gln Ala His Leu Val His Gly Val Gln Glu Gln Thr Phe
   465 470 475 480
   Ile Ala His Val Met Ala Phe Ala Ala Cys Leu Glu Pro Tyr Thr Ala
   485 490 495
   Cys Asp Leu Ala Pro Pro Ala Gly Thr Thr Asp
   500 505
<210> 5
   <211> 21
   <212> DNA
   <213> Bos taurus
<220>
   <221> misc_feature
   <223> Partial sequence from calf intestinal alkaline phosphatase (ciAP)
<400> 5
   cttcggcgtt cagtaacacg c 21
<210> 6
   <211> 24
<212> DNA
   <213> Bos taurus
<220>
   <221> misc_feature
   <223> Partial sequence from calf intestinal alkaline phosphatase (ciAP)
<400> 6
   cctctagatt atcagtcggg gatg 24
<210> 7
   <211> 21
   <212> DNA
   <213> Bos taurus
<220>
   <221> misc_feature
   <223> Partial sequence from calf intestinal alkaline phosphatase (ciAP)
<400> 7
   ggtcacgtct gtgatcaacc g 21
<210> 8
   <211> 21
   <212> DNA
   <213> Bos taurus
<220>
   <221> misc_feature
   <223> Partial sequence from calf intestinal alkaline phosphatase (ciAP)
<400> 8
   cggttgatca cagacgtgac c 21
<210> 9
   <211> 22
   <212> DNA
   <213> Bos taurus
<220>
   <221> misc_feature
   <223> Partial sequence from calf intestinal alkaline phosphatase (ciAP)
<400> 9
   gctttcgagg tgaatttcga cc 22

## Description

The present invention relates to a quick, simple and efficient *in vitro* method for generating mutated circular polynucleotide molecules. The method is based on providing a closed circular DNA polynucleotide template comprising a first gene of interest, providing fragments of a double-stranded DNA target polynucleotide comprising a second gene of interest, capable of hybridizing to the first gene of interest, wherein the fragments have free 3'OH ends and phosphorylated 5'-ends, generating single strands of both said template polynucleotide and said fragments of the target polynucleotide, annealing the fragments of the DNA target polynucleotide to the circular polynucleotide, elongating the fragments, e.g., by using a DNA polymerase, ligating the elongated fragments to each other, e.g., by using a DNA ligase. These steps result in a recombined and/or mutated circular daughter strand DNA. To further increase the frequency of mutation and/or recombination the steps of annealing, elongation and ligation are repeated 1 to 100 times. A multitude of mutated and/or chimaeric polynucleotides is easily obtained by this method. The invention also discloses methods for obtaining recombinant biomolecules based on such mutated polynucleotides.

There is a major interest in bio-molecules for medical, industrial and scientific applications (Powell, K.A., et al., Directed Evolution and Biocatalysis, Angew. Chem. Int. Ed. Engl. 40 (2001) 3948-3959) (Kirk, O., et al., Industrial enzyme applications, Curr. Opin. Biotechnol. 13 (2002) 345-351).

Some applications require bio-molecules with very special properties or features which do not exist or are very hard to find in nature. One way to generate such bio-molecules mimics the principle of natural evolution. Random mutants of a bio-molecule are generated and a mutant with the desired property is selected by an appropriate screening process. This kind of approach is termed "directed evolution" (Arnold, F.H., and Volkov, A.A., Directed evolution of biocatalysts, Current Opinion in Chemical Biology 3 (1999) 54-59).

Various techniques can be used to create these mutants (Vasserot, A.P., et al., Optimization of protein therapeutics by directed evolution, Drug Discov Today 8 (2003) 118-126) (Graddis, T.J., et al., Designing proteins that work using recombinant technologies, Curr. Pharm. Biotechnol. 3 (2002) 285-297) (Brakmann, S., Discovery of superior enzymes by directed molecular evolution, Chembiochem. 2 (2001) 865-871).

The methods used today to generate bio-molecules with desired properties can be roughly divided into techniques introducing point mutations and techniques in which similar but not identical polynucleotide sequences are recombined.

The first type of methods leads to mutated polynucleotides by introducing random point mutations into the sequence of interest, e.g., based on error prone polymerase chain reaction (PCR) (Cadwell, R.C., and Joyce, G.F., PCR Methods Appl. 2 (1992) 28-33), use of so-called mutator strains (Greener, A., et al., Methods Mol. Biol. 57 (1996) 375-385), or on treating the DNA with chemical mutagens (Deshler, J.O., Gen. Anal. Techn. Appl. 9 (1992) 103-106).

The second type of technique which leads to chimaeric polynucleotides comprises the recombination of genes or gene-fragments derived from different variants of a gene.

In WO 01/34835 techniques are described for the recombination of functional domains.

The most commonly used recombination method for optimization of bio-molecules is called "DNA Shuffling" (US 5,834,252; US 5,605,793; US 5,830,721; US 5,837,458; US 5,811,238). This method comprises the creation of double-stranded fragments from different double-stranded DNA templates wherein said different DNA templates contain homologous and heterologous areas. The reassembly of the random fragments is performed by a PCR-like reaction without further addition of primers. The occurrence of overlapping domains of the DNA sequences which can function as primers/templates to each other is essential for a successful reassembly. Due to the poor yield of the recombined DNA sequence after reassembly, it usually has to be amplified by an additional PCR reaction and afterwards it has to be cloned in an appropriate expression system. The gene products can then be transformed into appropriate host cells, expressed and then screened for an enhanced or new property.

Methodological alternatives to DNA shuffling are random priming recombination (RPR) (Shao, Z., et al., Nucleic Acids Res. 26 (1998) 681-683) and the staggered extension process (StEP)( Zhao, H., et al., Nat. Biotechnol. (1998) 16258-16261).

Site directed mutations can be achieved by the methods disclosed in WO 02/16606; WO 99/35281. These methods use circular DNA as a template and site directed mutations are introduced by use of specially designed primers already comprising point mutations.

WO 01/29211 also describes a recombination method. The template polynucleotide according to this method is not integrated into a circular DNA, making it necessary to insert the recombined DNA in a suitable expression system after recombination.

Another method for obtaining chimaeric genes is described in WO 00/09697. The method disclosed there achieves a larger diversity of recombined DNA molecules by using various types of linear assembly DNA matrices.

Despite the significant progress in the various methods leading to mutant and especially to chimaeric polynucleotides there is still a tremendous need to provide a method for generating chimaeric polynucleotides that would help to at least partially avoid the known drawbacks of the state of the art procedures.

The aim of the present invention was therefore to develop a simple and efficient DNA recombination method for obtaining chimaeric polynucleotides by reducing the number of working steps as much as much as possible while achieving a high level of diversity in the resulting chimaeric polynucleotides.

It has been found that the disadvantages of the methods known in the art can be at least partially overcome by the methods, host cells and recombinant bio-molecules according to the present invention.

### Summary of the invention

The present invention relates to a method for generating a mutated and/or chimaeric polynucleotide, the method comprising providing a closed circular DNA polynucleotide template comprising a first gene of interest, providing fragments of a double stranded DNA target polynucleotide comprising a second gene of interest, capable of hybridizing to the first gene of interest, said fragments having free 3'OH ends and phosphorylated 5'-ends, generating single strands of both said template polynucleotide and said fragments of the target polynucleotide, annealing said fragments of the target polynucleotide to said template polynucleotide, elongating the annealed fragments of the target polynucleotide by a DNA polymerase, ligating the nicks between the elongated fragments by using a DNA ligase thereby generating a chimaeric full length circular DNA molecule, and then using the said chimaeric full length circular DNA molecule repeatedly as a closed circular DNA template polynucleotide by again generating single strands, annealing, elongating and ligating as described before. Since this technology in some aspects resembles the very popular polymerase chain reaction the term polymerase chain chimaerization (PCC) is proposed.

A method for obtaining a transformed host cell by introducing a mutated and/or chimaeric polynucleotide obtained according to a method of the present invention is also disclosed.

Additionnally, a host cell containing a mutated and/or chimaeric polynucleotide produced by a method according to the present invention is disclosed.

A further embodiment concerns a method for obtaining a recombinant bio-molecule which comprises obtaining a mutated and/or chimaeric polynucleotide by use of a method according to this invention, transforming said mutated and/or chimaeric polynucleotide into an appropriate host, expressing the recombined gene, and screening for said recombinant bio-molecule.

### Description of the invention

In a first preferred embodiment the present invention relates to a method for generating a mutated and/or chimaeric polynucleotide, the method comprising providing a closed circular polynucleotide template comprising a first gene of interest, providing fragments of a double stranded DNA target polynucleotide derived from a second gene of interest capable of hybridizing to the first gene of interest said fragments having free 3'OH ends and phosphorylated 5'-ends, generating single strands of both said template polynucleotide and said fragments of the target polynucleotide, annealing said fragments of the target polynucleotide to said template polynucleotide, elongating the annealed fragments of the target polynucleotide by a DNA polymerase, ligating the nicks between the elongated fragments by using a DNA ligase thereby generating a chimaeric full length circular DNA molecule. The mutated and/or chimaeric full length circular DNA molecule may then be used repeatedly as a closed circular polynucleotide template in the next cycles of chimaerization by again generating single strands, annealing, elongating and ligating as described before. For convenience this method is referred to as a chimaerization method, although other advantageous mutants without a recombination event are also obtained by the inventive method.

The term "mutated and/or chimaeric gene" is used to indicate that mutated and/or chimaeric polynucleotides are obtained at a high frequency by using the method according to the present invention. As known in the art mutations are for example point mutations, deletions and insertions. A chimaeric polynucleotide in the sense of the present invention is any gene of interest comprising at least 15 consecutive polynucleotides derived from two different genes of interest. Thus although the major advantage of the present invention is the high frequency of chimaerization which is obtained, it also has the additional and welcome side effect that mutated polynucleotides are also frequently generated when using the chimaerization method of the present invention. The term "and/or" is used to indicate that polynucleotides are obtained comprising mutations alone, mutations as well as chimaeric sequences, or chimaeric sequences in the absence of further mutations.

Preferably the gene selected by a method according to the present invention is a chimaeric gene with or without additional mutations.

A "gene of interest" relates to any polynucleotide sequence exhibiting a property which can be selected or screened by any appropriate screening or selection procedure. The present invention also makes use of at least a "first" and a "second" gene of interest.

The term "a" in relation to the first or to the second gene of interest must not be understood as exclusively referring to a single gene. Rather the present invention can also be successfully applied using several different template as well as several different target genes. Preferably all these template and target genes represent variants of a single gene. It is, however, preferred to use three, two and/or only one template or target gene or genes, respectively.

Obviously the molar ratio of the template gene to fragments of a target gene will influence the degree of chimaerization achieved. Molar ratios (fragments/template) from 15-1000 are recommended, with ratios of 10-500, 20-400 and 30 to 300 being more preferred.

The first and the second gene of interest must be at least partially homologous in order to allow hybridization and annealing as required for the inventive method. The skilled artisan will have no problems in selecting appropriate pairs of genes for performing the method of this invention.

In order to obtain an adequate frequency of hybridization or annealing the template and the target gene must have a homology of at least 40 %. Preferably the homology between the two genes is at least 50 %. The homology is preferably higher and at least 60 % or at least 70 %. Most preferably, the homology is 80 % or more, as determined in the method described below.

The homology between the first and the second gene of interest is preferably analyzed by the PileUp program of the GCG Package, Version 10.2 (Genetics Computer Group, Inc.). PileUp creates a multiple sequence alignment using a simplification of the progressive alignment method of Feng, D.F., and Doolittle, R.F., J. Mol. Evol. 25 (1987) 351-360, and the scoring matrixes for identical, similar, or different amino acid residues are defined accordingly. This process begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. The final alignment is achieved by a series of progressive, pairwise alignments that include increasingly dissimilar sequences and clusters, until all sequences have been included in the final pairwise alignment.

For convenience sequence identity may also be assessed when comparing the template and the target gene. In such comparisons individual nucleotides at corresponding sequence positions are compared with one another. The sequence identity is preferably at least 40 % for at least one partial sequence of 30 nucleotides in length. More preferably the sequence identity is higher and at least 50 % or even more preferably at least 60 %.

Many related genes occurring in nature serve the same purpose in different individuals as well as in different species. By way of example, hemoglobin is used to transport oxygen in blood. Within the human species hemoglobin is known to be present with slightly different sequences (known as alleles), whereas hemoglobin in other mammals exhibits more differences on a sequence level. These genes thus represent naturally occurring variants of a single gene or gene locus. Preferably both a template and a target gene represent naturally occurring variants of a single gene.

As the skilled artisan will appreciate, any combination of template and target gene will be appropriate as long as fragments of the target gene hybridize to the template gene. The conditions of hybridization may be varied, e.g., by changing buffer conditions. According to the present invention annealing is preferably performed in a buffer consisting of 0.2M Tris-HCl, 0.02M MgSO₄, 0.1 M KCl, 0.1 M (NH₄)₂SO₄, 1 % Triton X-100, and 1 mg bovine serum albumin (BSA)/ml at pH 8.8. Any pair of template and target gene, which yields a sufficient number of annealing events in the first round of chimaerization in said buffer, is appropriate for performing the chimaerization method according to this invention.

The first gene of interest is provided as an integral part of a closed circular polynucleotide. As a matter of convenience the first gene of interest as comprised in a closed circular DNA polynucleotide is also referred to as a "template" gene.

Any type of circular polynucleotide (RNA or DNA) or even circular pseudopolynucleotides comprising nucleotide analogues like for example peptide nucleic acids (PNAs) may be used as a first polynucleotide. In an advantageous and preferred embodiment, the first polynucleotide comprising the template gene is a deoxyribonucleic acid (DNA). The closed circular polynucleotide is most preferably double-stranded DNA.

There are numerous methods for cloning a certain gene and inserting it in a suitable expression system which do not have to be recited here. For convenience it is mentioned that the skilled artisan is quite familiar with these methods which are summarized for example in Current Protocols in Molecular Biology Volume 1-4, Edited by Ausubel, F.M., et al., Massachusetts General Hospital and Harward Medical School by John Wiley & Sons, Inc.

The template gene can easily be amplified and at the same time methylated by transforming the plasmid into a suitable host strain, inoculating the plasmid-carrying strain for an appropriate time and isolating the plasmid from the cell culture. The use of a methylated circular polynucleotide provides an additional advantage. After the chimaerization is completed in a method according to the present invention the methylated DNA (i.e. the starting material) can be easily removed by using specific restriction enzymes which cut out only methylated DNA. As the initial template is methylated DNA and only non-methylated circular mutated and/or chimaeric DNA molecules are produced, the starting template can be eliminated by using an enzyme like DpnI that only cleaves methyl-DNA. The most preferred way to perform the inventive method thus comprises using a methylated closed circular DNA as the polynucleotide containing the first gene of interest.

Preferably the polynucleotide sequence of the first gene of interest is the full length polynucleotide for this gene. As the skilled artisan will appreciate, this gene may however, be shortened to represent only partial sequences of a gene. The minimum length required is a polynucleotide which exhibits a biological activity or function which can be identified by any appropriate screening or selection method.

The second gene of interest is also referred to as a "target" gene for convenience. The target gene must be available in a double-stranded form and preferably as DNA double-strands. Exponential amplification during the various rounds of chimaerization is only achieved if the fragments derived from the target gene comprise fragments of both the sense as well as of the anti-sense strand.

The target gene or polynucleotide is not provided as a full length continuous molecule but rather in a fragmented form. As the skilled artisan will appreciate, appropriate fragments of a double-stranded DNA polynucleotide may be generated by various means. The methods for providing fragments of a double-stranded target polynucleotide are not critical as long as care is taken that at least some of the fragments thus generated are capable of hybridizing to the first gene of interest with the additional requirement that the fragments provided must have a 3'OH and a phosphate residue at the 5'-end, respectively. Most preferred are methods leading to a random fragmentation of the target gene.

Random fragments can be obtained by numerous methods known to the skilled artisan. One possibility would be to amplify the DNA sequence of interest by PCR and randomly cut the PCR product by an endonuclease resulting in fragments with the required 3'OH and 5'phosphate ends. For example, digestion can be accomplished in a very short time with DNAse I and the degree of digestion can be monitored on an agarose gel. After the desired degree of fragmentation is achieved the reaction can be stopped, e.g., by heating the sample to 95°C for 5 minutes thus destroying the nuclease. The DNAse I can also be separated from the fragments by various methods i.e. using polymerase chain reaction (PCR) purification kits which are commercially available. Another possibility would be to use very short primers for random annealing in a PCR reaction with the second DNA sequence of interest as the template resulting in PCR products of different lengths and the 5' ends of the fragments thus obtained can then be phosphorylated with an appropriate kinase. Usually fragments with a length between 8 and 1000 base pairs (bp) should be used. In a preferred embodiment the majority (more than 50%) of the fragments used will be between 10 and 300 bp.

The terms annealing, elongating and ligating as used in the present invention have the meanings the skilled artisan attaches thereto.

The method according to the present invention may be repeated 1-100 times. Whereas in the first round of chimaerization only the closed circular polynucleotide template comprising the first gene of interest is present, an ever increasing number of already mutated and/or chimaeric closed circular DNA polynucleotides will be present in each of the following cycles which will again act as a template for the next cycle according to the present invention. The use of polynucleotides that are already mutated and/or chimaeric polynucleotides as templates in the consecutive rounds of chimaerization further increases the total number of different mutated and/or chimaeric molecules.

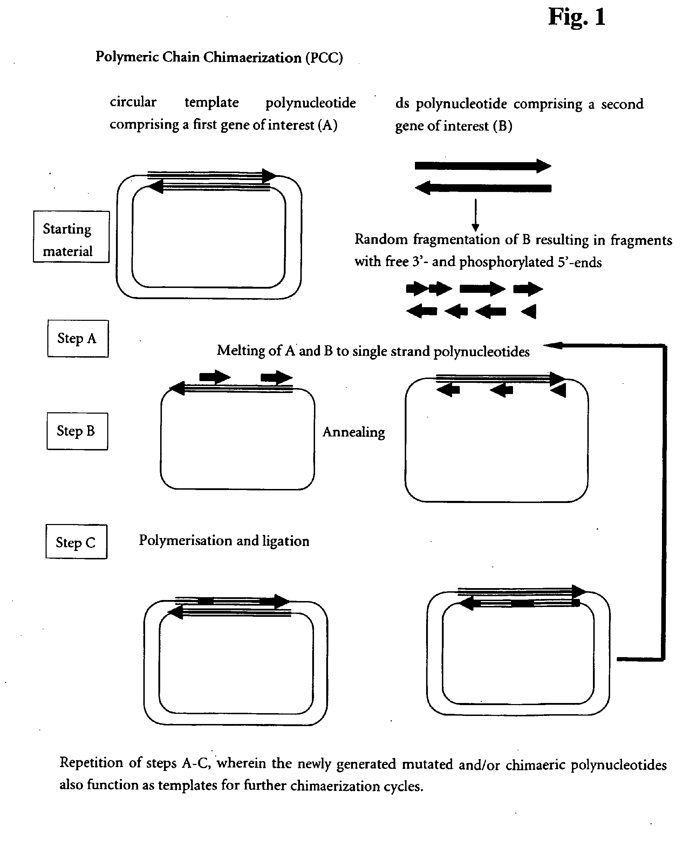

The method according to the present invention is further illustrated in Figure 1. In a first step single-chain molecules are generated for both the template as well as the target polynucleotide. In a second step single-stranded DNA fragments of a target gene or sequence of interest are annealed to a template polynucleotide which is incorporated in a circular closed single strand polynucleotide molecule. The annealed fragments are elongated by a DNA polymerase until the enzyme meets an already bound fragment. The DNA polymerase then drops of the DNA strand leaving a nick between the elongated 3'OH ends of a fragment and the 5'phosphate ends of a fragment. The gap or nick is closed by a DNA ligase which ligates the 3'OH-extension to the phosphorylated 5'-end of the next fragment bound. A double-stranded DNA comprising two closed circular DNA molecules is obtained as the final result of such a cycle of chimaerization. It is obvious to the skilled artisan that the number of annealed fragments determines the degree of chimaerization of the DNA sequences of interest. The method according to the present invention provides the major advantage that the double-stranded closed circular polynucleotide obtained in the first or more generally in a previous round of chimaerization can be directly used in a next round of chimaerization. In order to perform a next round of chimaerization the double strand obtained in the first round is melted and both single stranded circular molecules now function as a template polynucleotide in the next cycle of chimaerization. Preferably two to fifty cycles of chimaerization as described above are performed. This leads to an ever increasing number of recombinations resulting in a great diversity of mutated and/or chimaeric polynucleotides. Between 15 and 35 cycles of chimaerization are most preferably used.

The efficient and convenient method of chimaerization according to the present invention can easily be performed in a single mixture of reagents which contains all required reactants. Details of preferred reactants are given further below and in the example section. If required the inventive method can also be performed in steps, e.g. fragments of the target polynucleotide could be added at the beginning and again after an appropriate number of chimaerization cycles. Such and similar modifications are of course possible within the scope of the present invention.

The chimaerization can be achieved by carrying out a PCR-like reaction using a closed circular polynucleotide comprising the template gene and fragments of the target gene with both orientations as primers. Preferably the reaction is carried out at elevated temperatures. The use of a thermo-stable DNA polymerase and of a thermo-stable DNA ligase, like DNA polymerases and ligases obtained from organisms like *Pyrococcus furiosus, Thermos aquaticus,* etc. is also preferred. It is also advisable and preferable to use a DNA polymerase with proofreading activity to ensure that unwanted polymerization errors beyond the DNA sequence of interest are avoided. This is also advantageous for retaining an intact expression system.

As described above the product of a chimaerization cycle functions as a template for the remaining fragments in the reaction mixture and the chimaerization cycle can be repeated. This means that as in a normal PCR reaction an exponential amplification of the mutated and/or chimaeric DNA sequences is achieved and furthermore that the number of polynucleotides is exponentially amplified. The ratio of starting template to newly generated DNA increases in favor of the chimaeric molecule in each cycle. This also substantially reduces the risk or likelihood of obtaining parental clones after transformation of the mutated and/or chimaeric polynucleotides into an appropriate host cell.

A standard PCR-like chimaerization cycle according to the present invention for example utilizes the following reagents: a buffer system in which the chosen polymerase and ligase work, dNTP's, polymerase, ligase, ATP (or another cofactor for ligase), template and fragments of the target polynucleotide; and comprises the following steps: incubation at 72°C to ligate undesired nicks in the circular template, 30 seconds at 95°C to melt the double-stranded DNA into single-stranded DNA, 1 minute at 50°C to anneal the fragments to the template, 8 minutes at 72°C to elongate the fragments by DNA polymerase, 30 seconds at 95°C to remove bound DNA polymerase from the template and 8 minutes at 72°C to ligate the fragments to each other and to eliminate any nicks. The chimaerization cycle is preferably repeated about 20 to 25 times. Finally the reaction mixture is cooled to 4°C.

As the described method is based on the use of closed circular polynucleotides and results predominantly in closed circular chimaeric DNA molecules, it is evident that it is advantageous to use plasmids as closed circular DNA template molecules. Such a plasmid preferably comprises a suitable expression system for the DNA sequence or gene of interest. The template polynucleotide is preferably a double-stranded closed circular plasmid comprising an origin of replication, a promoter for gene expression, a gene for selection and a first gene of interest. As the skilled artisan will readily appreciate preferably only that part of the polynucleotide sequence which comprises the gene of interest is recombined and the rest of the DNA sequence, i.e. the expression system or plasmid part of the sequence is left untouched. Two or more different plasmids containing the DNA sequence of interest may also be used in a method according to this invention.

The working steps usually needed by the other methods known from the art, e.g. requiring the insertion of the newly mutated and/or chimaeric DNA molecules into an appropriate expression system are not necessary in the case of the mutated and/or chimaeric DNA polynucleotides produced by a method according to this invention are already incorporated in an expression system. Hence additional steps such as the preparation of vectors and insertion using suitable restriction enzymes and ligation of the later are avoided. This makes the inventive method much more convenient and simple to use.

The result of the chimaerization PCR i.e. PCC can be monitored by agarose gel electrophoresis. A polynucleotide of the desired size (identical to the template) should be visible on the agarose gel.

The present invention is also useful for a method for obtaining a transformed host cell by introducing a mutated and/or chimaeric polynucleotide obtained according to a method of the present invention into said host. Bacteria like *E.coli* or *Bacillus* strains are preferred as host strains. Such transformations have been found to be quite successful and produce a high yield of transformants.

The mutated and/or chimaeric circular DNA obtained in a method according to the present invention can be subsequently transformed into an appropriate host cell without any further working steps. As described above, this transformation can optionally incorporate a step for removing a methylated template polynucleotide. However, this step also does not require further purification. It is therefore preferable to directly use the mutated and/or chimaeric polynucleotides obtained in a method according to this invention without prior purification to transform a host cell.

Of course a host cell obtained by transformation with a mutated and/or chimaeric polynucleotide obtained by the method of the present invention is also useful.

The present invention also relates to a method for obtaining a recombinant bio-molecule, the method comprising: obtaining a mutated and/or chimaeric polynucleotide by use of a method according to the present invention, transforming said mutated and/or chimaeric polynucleotide into an appropriate host, expressing the recombined gene, and screening for said recombinant bio-molecule.

After isolation of individual clones, inoculation and expression of the mutated and/or chimaeric gene products the new bio-molecules can be screened in order to identify clones containing a recombinant gene with the desired property.

Screening may be performed for any selectable property. Preferably the bio-molecule is an expression vector and expression vectors that lead to a higher protein yield are selected. The bio-molecule is most preferably a polypeptide and the selection process is for example simply based on a selectable property of such a polypeptide. Such a recombinant polypeptide is most preferably an enzyme. As the skilled artisan will appreciate, different enzymatic properties are quite easily selected for once the new (mutated and/or chimaeric) polynucleotide has become available and has been expressed as described above.

As the skilled artisan will readily appreciate the methods disclosed herein are quite advantageous for various reasons, e.g. they obviate the necessity for an amplifying PCR reaction that is still required by state of the art methods especially in order to generate mutated and/or chimaeric DNA molecules at a reasonable frequency and no purification step prior to transformation is necessary. It is also quite obvious that by omitting the amplification PCR no pre-selection of mutated and/or chimaeric polynucleotide occurs. This results in a larger diversity of mutated and/or chimaeric polynucleotides entering into transformation which also results in a larger diversity of genetic variants for screening and selection.

The following examples, references, sequence listing and figures are provided to aid the understanding of the present invention, the true scope of which is set forth in the appended claims.

### Description of the Figures

- **Figure 1**: **Schematic of a method according to the present invention**
In Figure 1 the inventive method using a double-stranded closed circular polynucleotide comprising a first gene of interest (striped arrow) and fragments derived from a double-stranded target DNA (bold arrow) is shown. The polynucleotides are provided and mixed with all reagents required, then the steps of melting, annealing, polymerization and ligation are performed.
- **Figure 2**: **Amino acid sequences for hAP and ciAP**
The amino acid sequence for calf intestinal alkaline phosphatase is shown in the first row and the corresponding sequence of human placental alkaline phosphatase is shown in the second row.
- **Figure 3**: **Fragmentation of target DNA**
The results of target DNA fragmentation with DNAse I according to Example 1 are shown in Figure 3.
- **Figure 4**: **Nucleic acids obtained after 20 cycles of chimaerization**
5 µl of sample, obtained as described in Example 1, were applied to a 1 % agarose gel. After electrophoresis the DNA bands were stained with ethidium bromide. Stained bands are shown.

### Example 1

### Chimaerization of human placental alkaline phosphatase (hpAP) with calf intestinal alkaline phosphatase (ciAP)

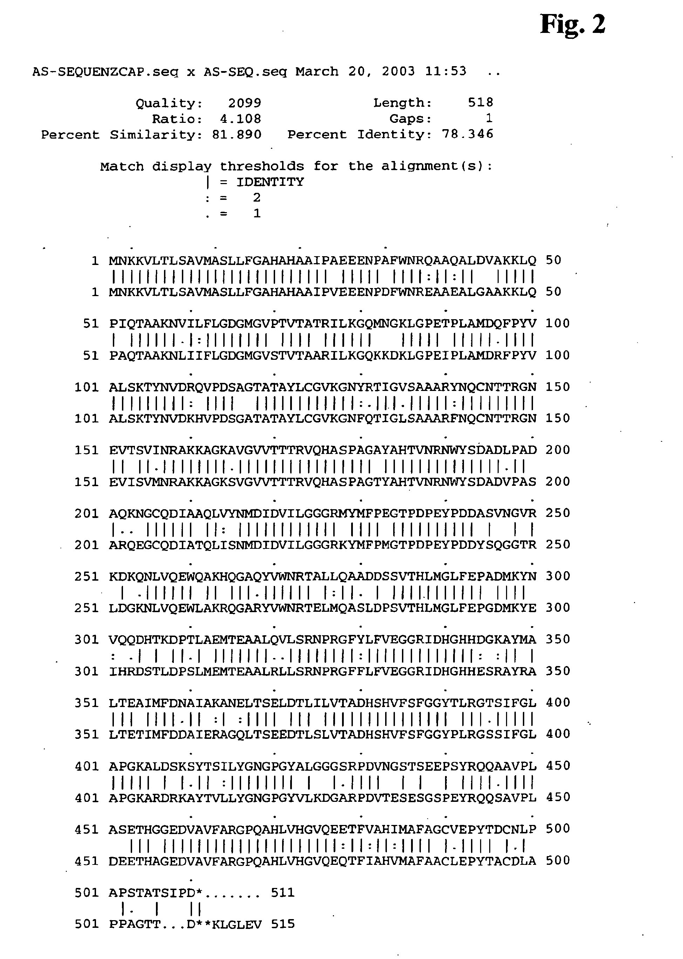

The genes of ciAP and hpAP exhibit a homology of about 80 % to each other (see SEQ ID NO: 1 and 3, respectively). Both the amino acid sequences are shown in Figure 2 and in SEQ ID NOs 2 and 4, repectively. The distribution of homologous and heterologous areas is nearly random over the whole gene making it easy to monitor the degree of chimaerization of both the genes.

The two genes were cloned into the plasmid pkk 177 with an ampicillin resistance gene where they were under the control of a pmgl promoter (see patent application WO88/09373) and transformed into the *E.coli* strain XL-blue-MRF'.

For the following experiment hpAP was used as a first gene of interest.

The random fragments of the target gene were generated from the ciAP gene.

### Template gene (plasmid):

The *E.coli* strain carrying the plasmid with the hpAP gene was inoculated over night at 37°C. The plasmid was isolated using a Roche High Pure Plasmid Isolation Kit Id. 1754777 and the concentration determined by UV at 260 nm. A solution of 5 ng DNA/µl water was prepared.

### Target gene (fragments):

An aqueous solution containing the plasmid carrying the ciAP gene was prepared using the same method as for the template. The ciAP gene was amplified by PCR using suitable primers (see SEQ ID NO: 5 and 6, respectively).

The resulting PCR product was purified using a Roche PCR purification Kit and the DNA concentration was determined.

### Fragmentation:

The ciAP gene was subjected to DNAse I digestion. The following preparation was made:
30 µl ciAP (100 ng DNA/µl water)
1.5 µl DNAse I (0.1 U/µl; Roche Cat. No. 776785 diluted with 1x Puffer Cat. No.1417991)
8 µl buffer (10 x Puffer Cat. No.1417991)
40.5 µl water

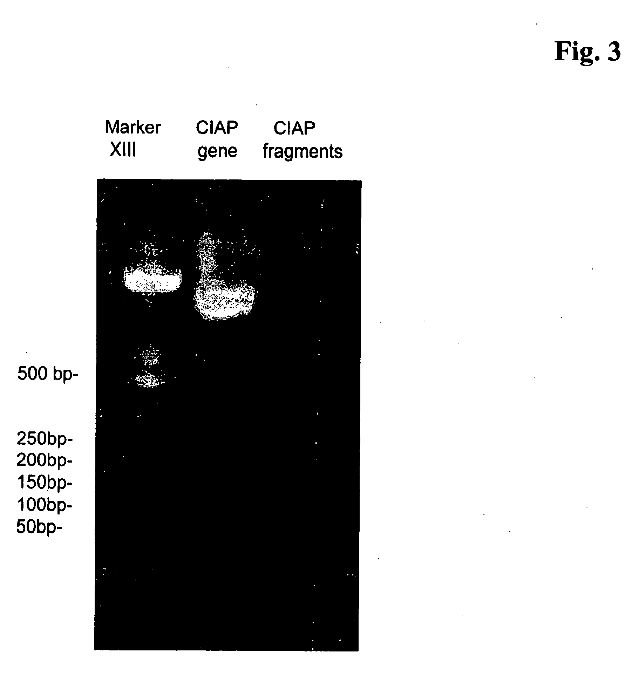

After 10 minutes incubation at room temperature the preparation was quickly frozen on dry ice to stop DNAse I digestion. 5 µl sample were analyzed on a 1 % agarose gel to monitor digestion (see Figure 3).The cloud of fragments proved that the majority of fragments was about 50 bp in size. DNAse I was inactivated by heating the remaining preparation at 95°C for 10 minutes. The DNA was purified by the Roche PCR purification Kit and the DNA concentration was determined at 260 nm.

### Chimaerization-PCR (PCC):

| | **neg. control (without fragments)** | **PCC-sample** |
|---|---|---|
| hpAP template 5ng/µl | 2 µl | 2 µl |
| ciAP fragments 19 ng/µl | - | 10 µl |
| 2.5U/µl Pfu polymerase | 1 µl | 1 µl |
| 10 x Pfu polymerase buffer | 5 µl | 5 µl |
| 4 U/µl Pfu ligase | 1 µl | 1 µl |
| 40 mM dNTP | 1 µl | 1 µl |
| 5µM/µl ATP | 1 µl | 1 µl |
| water | 39 µl | 29 µl |

2.5U/µl Pfu polymerase from Stratagene Cat. No. 600252
10 x Pfu polymerase buffer from Stratagene Cat. No. 600252
4 U/µl Pfu ligase from Stratagene Cat. No. 600191
40 mM dNTP = 10 mM dATP, 10 mM dCTP, 10 mM dTTP, 10 mM dGTP
5 µM/µl ATP from Roche Cat.No.1140965

Temperature program:
a) 10 minutes 72°C
b) 30 seconds 95°C
c) 1 minute 55°C
d) 8 minutes 72°C
e) 30 seconds 95°C
f) 8 minutes 72°C
g) 20-fold repetition of steps b) to f)
h) 4°C

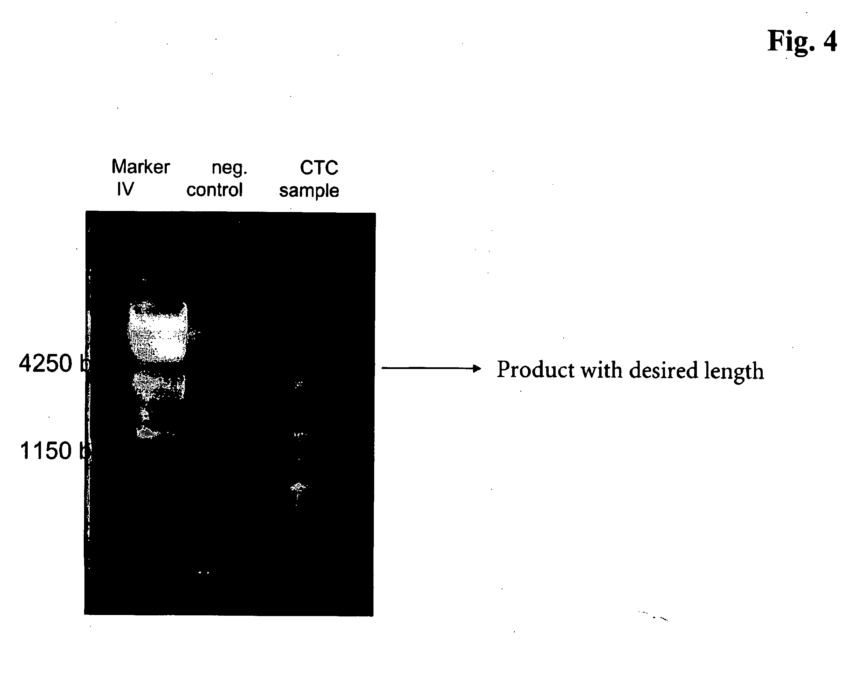

After the thermo-cycling 5 µl of the PCC-PCR products were applied to a 1 % agarose gel (see Figure 4).

Results: The PCC-sample shows a band having the desired size, whereas the neg. control shows none.

### Example 2

### Transformation

Transformation was done by electroporation of both samples PCC and neg. control respectively into the *E.coli* XL-MRF' strain (Stratagene Cat. No. 200158). Afterwards 100 µl of the PCC-preparation and 1 ml of the neg. control were plated out on an LB agar plate containing 100 µg/ml ampicillin. Cells were incubated over night at 37°C.

The results of this transformation experiment in terms of the number of growing clones are given in the following Table:

| | **Neg. control-sample** | **PCC-sample** |
|---|---|---|
| Clones | 0 | 640 |

20 clones where randomly picked from the PCC-sample plate and separately inoculated in LB-amp medium overnight at 37°C. The plasmids of the 20 samples were isolated using a Roche High Pure Plasmid Isolation Kit Id. 1754777 and sequenced using an ABI Prism Dye Terminator Sequencing Kit and ABI 3/73 and 3/77 sequencer (Amersham Pharmacia Biotech). The sequencing primers used were all based on the ciAP gene (see SEQ ID NOs: 5, 7, 8 and 9, respectively).

### Example 3

### Testing clones for AP activity

The AP activity was determined as follows: 90 µl cell suspension of the overnight culture was mixed with 10 µl of B-PER (Bacterial Protein Extraction Reagent, Pierce Cat. No. 78248) to disrupt the cells. 50 µl of this mixture were added to 90 µl reagent (Roche Cat. No. 2173107). Active AP releases p-nitrophenol which can be monitored by a photometer at 405 nm. An *E.coli* XL-MRF' strain cell suspension without hpAP or ciAP-plasmid was used as a neg. control.

### Example 4

### Mutant and chimaeric clones obtained

The results of AP activity testing and corresponding clone analysis by sequencing are given in the following table:

| **Clone** | **Chimaerization** | **Mutation** | **Activity in ΔA/min** | **Remarks** |
|---|---|---|---|---|
| 1 | ciAP to hpAP at 478 | V69A | 0 | - |
| 2 | Whole protein as ciAP | - | 0,033 | - |
| 3 | ciAP to hpAP at 130 and from hpAP to ciAP at 165 | - | 0 | Primer SEQ ID NO: 7 did not bind, 32 aas not sequenced |
| 4 | Whole protein as hpAP | Q41E | 0 | Primers SEQ ID NO: 7 and SEQ ID NO: 8 did not bind, 28 aas not sequenced |
| 5 | ciAP to hpAP at 508 | S136I, S507T | 0,006 | - |
| 6 | Whole protein as ciAP | D200N, Q202H | 0,006 | - |
| 7 | Whole protein as ciAP | Deletion from 497 to the end (6 amino acids) | 0,005 | - |
| 8 | Whole protein as ciAP | T436A, G403S | 0,016 | - |
| 9 | Whole protein as ciAP | D218G | 0 | - |
| 10 | ciAP to hpAP at 478 | I375F | 0 | - |
| 11 | ciAP up to 150 then random sequence | | 0 | - |
| 12 | ciAP to hpAP at 156 | K104R | 0,07 | Primers SEQ ID NO: 7 and SEQ ID NO: 8 did not bind; 28 aas not sequenced |
| 13 | Whole protein as ciAP | - | 0,013 | - |
| 14 | Whole protein as ciAP | K203, E438K, 1497S | 0,004 | - |
| 15 | Whole protein as ciAP | G128D | 0 | - |
| 16 | ciAP to hpAP at 37 and from hpAP to ciAP at 43 | Insertion at 443 of 23 amino acids (doubling of 23aa ciAP sequence at 443) | 0 | - |
| 17 | Whole protein as ciAP | - | 0,007 | - |
| 18 | no data | no data | 0 | - |
| 19 | no data | no data | 0 | - |
| 20 | ciAP to hpAP at 478 | M356V | 0,015 | - |

It has to be noted that hpAP is the starting template. Numbers refer to amino acid positions in the AP enzyme. Expressions like V69A mean an amino acid exchange at position 69 from V (valine) to A (alanine). The alignment of the sequences on an amino acid basis is shown in Figure 2.

The above data show that chimaerization occurred to almost any desired extent. The mutant spectrum ranges from no gene mixture at all (hpAP) to a complete reassembly of the fragmented ciAP gene.

Of the 20 clones isolated, 50 % showed alkaline phosphatase activity, also including mutants with mixed DNA. In fact the clone with the highest activity (No. 12) is a chimaera. Point mutations, insertions and deletions were observed in addition to the chimaerization further extending the pool of novel polynucleotides on which selection for novel or improved properties can be based.

### List of References

Arnold, F.H., and Volkov, A.A., Curr. Opin. Chem. Biol. 3 (1999) 54-59
Ausubel, F.M., et al., Massachusetts General Hospital and Harward Medical School by John Wiley & Sons, Inc.
Brakmann, S., Chembiochem. 2 (2001) 865-871
Cadwell, R.C., and Joyce, G.F., PCR Methods Appl. 2 (1992) 28-33
Deshler, J.O., Gen. Anal. Techn. Appl. 9 (1992) 103-106
Feng, D.F., and Doolittle, R.F., J. Mol. Evol. 25 (1987) 351-360
Graddis, T.J., et al., Curr. Pharm. Biotechnol. 3(2002) 285-297
Greener, A., et al., Methods Mol. Biol. 57 (1996) 375-385
Kirk, O., et al., Curr. Opin. Biotechnol. 13(2002) 345-351
Powell, K.A., et al., Angew. Chem. Int. Ed. Engl. 40 (2001) 3948-3959
Shao, Z., et al., Nucleic Acids Res. 26 (1998) 681-683
US 5,605,793
US 5,811,238
US 5,830,721
US 5,834,252
US 5,837,458
Vasserot, A.P., et al., Drug Discov. Today 8 (2003) 118-126
WO 00/09697
WO 01/29211
WO 01/34835
WO 02/16606
WO 99/35281
Zhao, H., et al., Nat. Biotechnol. 16 (1998) 258-261

## Claims

1. A method for generating a mutated and/or chimaeric polynucleotide, the method comprising:
a) providing a closed circular polynucleotide template comprising a first gene of interest,
b) providing fragments of a double-stranded DNA target polynucleotide derived from a second gene of interest, capable of hybridizing to the first gene of interest, said fragments having free 3'OH ends and phosphorylated 5'-ends,
c) generating single strands of both said template polynucleotide and said fragments of the target polynucleotide,
d) annealing said fragments of the target polynucleotide to said template polynucleotide,
e) elongating the annealed fragments of the target polynucleotide by DNA polymerase,
f) ligating the nicks between the elongated fragments by using a DNA ligase thereby generating a chimaeric full length circular DNA molecule, and
g) using the product obtained in f) and repeating steps c) to f) 1 to 100 times.

2. The method according to claim 1, wherein a thermo-stable DNA polymerase and a thermo-stable DNA ligase are used.

3. The method according to claim 1 or 2, wherein the gene of interest comprised in said template polynucleotide and the gene of interest comprised in said target polynucleotide are variants of a single gene.

4. The method according to any of claims 1 to 3, wherein two or more variants of a gene of interest are comprised in a template polynucleotide and/or in a target polynucleotide.

5. The method according to any of claims 1 to 4, wherein said template polynucleotide is a double-stranded closed circular plasmid comprising an origin of replication, a promoter for gene expression, a gene for selection and a first gene of interest.

6. A method for obtaining a recombinant bio-molecule, the method comprising:
a) obtaining a mutated and/or chimaeric polynucleotide by use of a method according to any of claims 1 to 5,
b) transforming said mutated and/or chimaeric polynucleotide into an appropriate host,
c) expressing the recombined gene, and
d) screening for said recombinant bio-molecule.

7. The method according to claim 6, wherein said bio-molecule is an enzyme.

## Patentansprüche

1. Verfahren zur Erzeugung eines mutierten und/oder chimären Polynukleotids, wobei man in dem Verfahren:
a) eine ein erstes interessierendes Gen umfassende geschlossene zirkuläre Polynukleotidmatrize bereitstellt,
b) Fragmente eines von einem zweiten interessierenden Gen abgeleiteten doppelsträngigen DNA-Zielpolynukleotids bereitstellt, die zur Hybridisierung an das erste interessierende Gen fähig sind, wobei die Fragmente freie 3'-OH-Enden sowie phosphorylierte 5'-Enden aufweisen,
c) Einzelstränge sowohl des Matrizenpolynukleotids als auch der Fragmente des Zielpolynukleotids erzeugt,
d) die Fragmente des Zielpolynukleotids in einer Annealing-Reaktion an das Matrizenpolynukleotid anlagert,
e) die angelagerten Fragmente des Zielpolynukleotids mittels DNA-Polymerase verlängert,
f) die Lücken zwischen den verlängerten Fragmenten unter Verwendung einer DNA-Ligase ligiert, wodurch ein chimäres zirkuläres Vollängen-DNA-Molekül erzeugt wird, und
g) mit dem in f) erhaltenen Produkt die Schritte c) bis f) 1 bis 100 mal wiederholt.

2. Verfahren nach Anspruch 1, wobei eine temperaturstabile DNA-Polymerase sowie eine temperaturstabile DNA-Ligase verwendet werden.

3. Verfahren nach Anspruch 1 oder 2, wobei es sich bei dem in dem Matrizenpolynukleotid enthaltenen interessierenden Gen und dem in dem Zielpolynukleotid enthaltenen interessierenden Gen um Varianten eines einzigen Gens handelt.

4. Vefahren nach einem der Ansprüche 1 bis 3, wobei mindestens zwei Varianten eines interessierenden Gens in einem Matrizenpolynukleotid und/oder in einem Zielpolynukleotid enthalten sind.

5. Verfahren nach einem der Ansprüche 1 bis 4, wobei es sich bei dem Matrizenpolynukleotid um ein einen Replikationsursprung, einen Promotor zur Genexpression, ein Gen zur Selektion sowie ein erstes interessierendes Gen umfassendes doppelsträngiges geschlossenes zirkuläres Plasmid handelt.

6. Verfahren zur Gewinnung eines rekombinanten Biomoleküls, wobei man in dem Verfahren
a) ein mutiertes und/oder chimäres Polynukleotid unter Verwendung eines Verfahrens nach einem der Ansprüche 1 bis 5 gewinnt,
b) das mutierte und/oder chimäre Polynukleotid in einen entsprechenden Wirt transformiert,
c) das rekombinierte Gen exprimiert und
d) ein Screening auf das rekombinante Biomolekül durchführt.

7. Verfahren nach Anspruch 6, wobei es sich bei dem Biomolekül um ein Enzym handelt.

## Revendications

1. Procédé pour générer un polynucléotide muté et/ou chimérique, le procédé comprenant :
a) la fourniture d'une matrice de polynucléotide circulaire fermée comprenant un premier gène d'intérêt,
b) la fourniture de fragments d'un polynucléotide cible d'ADN double brin dérivé d'un second gène d'intérêt, susceptible de s'hybrider au premier gène d'intérêt, lesdits fragments ayant des extrémités 3'OH libres et des extrémités 5' phosphorylées,
c) la génération de brins simples tant dudit polynucléotide matrice que desdits fragments du polynucléotide cible,
d) la condensation desdits fragments du polynucléotide cible audit polynucléotide matrice,
e) l'élongation des fragments condensés du polynucléotide cible par l'ADN polymérase,
f) la ligature des coupures simple brin entre les fragments allongés au moyen d'une ADN ligase, générant de la sorte une molécule d'ADN circulaire pleine longueur chimérique, et
g) l'utilisation du produit obtenu en f) et la répétition des étapes c) à f) de 1 à 100 fois.

2. Procédé selon la revendication 1, dans lequel une ADN polymérase thermostable et une ADN ligase thermostable sont utilisées.

3. Procédé selon la revendication 1 ou 2, dans lequel le gène d'intérêt compris dans ledit polynucléotide matrice et le gène d'intérêt compris dans ledit polynucléotide cible sont des variants d'un gène unique.

4. Procédé selon l'une quelconque des revendications 1 à 3, dans lequel deux ou plusieurs variants d'un gène d'intérêt sont compris dans un polynucléotide matrice et/ou dans un polynucléotide cible.

5. Procédé selon l'une quelconque des revendications 1 à 4, dans lequel ledit polynucléotide matrice est un plasmide circulaire fermé double brin comprenant une origine de réplication, un promoteur pour l'expression d'un gène, un gène pour la sélection et un premier gène d'intérêt.

6. Procédé pour l'obtention d'une biomolécule recombinante, le procédé comprenant :
a) l'obtention d'un polynucléotide muté et/ou chimérique grâce à l'utilisation d'un procédé selon l'une quelconque des revendications 1 à 5,
b) la transformation dudit polynucléotide muté et/ou chimérique dans un hôte approprié,
c) l'expression du gène recombiné, et
d) le criblage de ladite biomolécule recombinante.

7. Procédé selon la revendication 6, dans lequel ladite biomolécule est une enzyme.
